Europäisches Patentamt

European Patent Office

Office européen des brevets

(11) Publication number: **0 404 416** .

**A1**

# EUROPEAN PATENT APPLICATION

(21) Application number: **90306347.7**

(22) Date of filing: **11.06.90**

(51) Int. Cl.⁵: **B01D 69/14, B01D 71/82, C07C 7/144**

(30) Priority: **21.06.89 US 369126**

(43) Date of publication of application:
**27.12.90 Bulletin 90/52**

(84) Designated Contracting States:
**AT BE CH DE DK ES FR GB GR IT LI NL SE**

(71) Applicant: **EXXON CHEMICAL PATENTS INC.**
**200 Park Avenue**
**Florham Park New Jersey 07932(US)**

(72) Inventor: **Andison, David Douglas**
**121 Pearl Street**
**Sarnia, Ontario N7T5G6(CA)**

(74) Representative: **Northover, Robert Frank et al**
**Exxon Chemical Limited Exxon Chemical**
**Technology Centre PO Box 1**
**Abingdon Oxfordshire, OX13 6BB(GB)**

(54) **Transition metal exchanged ionomer membrane for hydrocarbon separation.**

(57) Process for preparing ion exchange membrane by drying ionomer having transition metal counterions and forming there from a configuration suitable for permselective applications, ion exchange membrane prepared by the process, and a method for separating an aliphatically unsaturated hydrocarbon from a hydrocarbon mixture using the ion exchange membrane.

EP 0 404 416 A1

# TRANSITION METAL-EXCHANGED IONOMER MEMBRANE FOR HYDROCARBON SEPARATION

This invention relates a process for preparing an ion exchange membrane and its use for separating aliphatically unsaturated hydrocarbons from hydrocarbon mixtures.

Aliphatically unsaturated hydrocarbons are significant raw materials for the preparation of chemical intermediates such as alcohols, organic acids and esters, and other compounds. These intermediates are used for the manufacture of detergents, engine oil lubricants, pesticides and other agricultural products, and plasticizers. In view of the importance of aliphatically unsaturated hydrocarbons, efforts have been made to develop efficient and relatively inexpensive means for separating these components from various hydrocarbon mixtures derived from refined petroleum or petrochemical process streams. Conventionally, the separation has been carried out by using cryogenic distillation methods, but these methods require high investment and operating costs.

Liquid barrier permeation techniques have been developed for the separation of aliphatically unsaturated hydrocarbons from mixtures as an alternative to conventional methods. U.S. Patent 3,758,605 discloses disposing a liquid barrier containing an aqueous solution of a complexing metal within a hydrophilic semipermeable membrane. The complexing metal first complexes with desired aliphatically unsaturated hydrocarbon components of the mixture to be separated upon contact with the membrane, and then reversibly dissociates from the desired components at the conditions which exist at the discharge of the membrane. Unfortunately, these liquid barrier membranes lack mechanical strength and physical integrity, especially when operated at conditions requiring a significant pressure drop across the membrane, e.g., pervaporation or perstraction conditions. In addition, active complexing agent is continuously removed during operation, which results in a steadily decreasing membrane selectivity.

In an attempt to overcome the deficiencies associated with liquid barrier membranes, U.S. Patent 4,318,714 describes preparing an ion exchange membrane having counterions which reversibly complex with olefins. The '714 patent discloses first casting an ionomer solution to prepare an ion exchange membrane film, and then immersing the ion exchange membrane in an aqueous solution of a suitable complexing agent metal salt, such as silver nitrate, Since the complexing agent is ionically bound to the membrane, it is not removed from the membrane to the extent associated with the liquid barrier membranes, and therefore overcomes one of the significant deficiencies with such membranes. However, the contacting of the ion exchange membrane with the aqueous solution of the complexing agent to prepare the desired membrane causes the membrane to swell. This swelling in turn causes the membrane to be sensitive to drying under certain conditions during operation, e.g., under pervaporation conditions, and more importantly, to exhibit poor selectivity when ultra-thin membranes are prepared. The preparation of ultra-thin membranes with good selectivity is desirable because membrane flux, which is the rate of permeation through the membrane, typically increases as the thickness of the discriminating membrane film decreases.

In view of the deficiencies of the prior art, a process is needed for preparing a membrane for the separation of aliphatically unsaturated hydrocarbons from hydro-carbon mixtures under a wide range of operating conditions and membrane configurations. More specifically, it would be desirable to prepare an ion exchange membrane which exhibits good selectivity for the desired separation at varying membrane thicknesses and under a wide range of operating conditions.

## Summary of the Invention

In one aspect, the invention is a process for preparing a semipermeable ion exchange membrane. The process comprises the steps of a) contacting an ionomer with an aqueous solution of a transition metal salt under conditions sufficient to exchange a plurality of counter-ions associated with ionic groups of the ionomer for transition metal ions of the transition metal salt, b) drying the transition metal-exchanged ionomer thereby removing a substantial amount of water from the ionomer, and c) forming from the dried ionomer a configuration which is operable for permselective applications.

In another aspect of the invention, the invention is a method of separating an aliphatically unsaturated hydrocarbon from a hydrocarbon mixture. The method comprises the steps of a) contacting a first major surface of the semipermeable ion exchange membrane described above with the hydrocarbon mixture, and then b) withdrawing from a second major surface of the membrane a permeate with an increased concentration of the aliphatically unsaturated hydrocarbon relative to the concentration of the aliphatically unsaturated hydrocarbon in the hydrocarbon mixture.

Surprisingly, ion exchange membranes of varying membrane thicknesses, particularly ultra-thin membranes, prepared by the process of this invention exhibit good selectivity for the separation of aliphatically unsaturated hydrocarbons from hydrocarbon mixtures. Such membranes can be operated over a wide range of operating conditions.

The invention represents an efficient and inexpensive means for carrying out separations of aliphatically unsaturated hydrocarbons from hydrocarbon mixtures.

Detailed Description of the Invention

For purposes of describing this invention, an ionomer is an ionically-bound polymer, advantageously an aromatic polymer having ionic groups bound to a plurality of aromatic rings on the polymer, wherein each ionic group is associated with a counterion. Preferably, the aromatic polymer is functionalized with anionic groups capable of exchanging cations, and swells slightly in water to promote intimate contact between the polymer and the aqueous transition metal salt solution.

Examples of suitable polymers from which the ionomers of this invention are derived include poly (phenylene oxide), polystyrene, polyacrylic acid, and perfluorinated polymers sold by DuPont under the tradename Nafion. The preferred ionically-bound groups are sulfonate, carboxylate and phosphonate groups. Methods for functionalizing the polymers with the ionically-bound groups are well known in the art, as evidenced by the discussion of ion exchange in Kirk-Othmer, Encyclopedia of Chemical Technology, Volume 13, 3rd Edition, John Wiley & Sons, pp. 678-705, 1981. Preferably, the ionomer has an ion exchange capacity of at least 0.5 meq/g, more preferably between about 2.0 and 3.5 meq/g. The ionomer is preferably sulfonated, and the most preferred ionomer is a sulfonated poly(phenylene oxide).

The transition metal salt must be capable of dissociating in water to form transition metal ions, and in addition, the transition metal ions must be capable of selectively and reversibly complexing with at least one aliphatically unsaturated hydrocarbon to facilitate the separation of such hydrocarbon from a hydrocarbon mixture. Useful transition metals from which the transition metal ions are derived are those of the first transition series having atomic numbers from 21 to 29, such as chromium, copper, manganese, nickel and iron. Other useful metals include the second and third transition series, i.e., having atomic numbers from 39 to 47 or 57 to 79, such as molybdenum, tungsten, and rhenium, as well as mercury. The preferred metals are the noble metals such as silver, gold, platinum and palladium. The most preferred transition metal is silver.

The ionomer and the aqueous transition metal salt solution may be contacted in such a manner as to promote the intimate contact of the ionomer with the solution to facilitate the exchange of ions. The simplest method for accomplishing intimate contact is by immersing the ionomer into the transition metal salt solutions with or without mixing, although any other method can be used. The concentration of transition metal salt in solution can vary considerably, but should be an amount at least sufficient to provide the membrane with an adequate complexing rate to achieve the desired separation. The contact can occur at room temperature, and the desired conditions for carrying out the exchange can be readily determined emperically by one skilled in the art.

After ion exchange is complete, the ionomer is separated from the aqueous phase. Preferably, residual transition metal salt is removed by washing the ionomer with water.

Drying can be carried out under any means suitable for removing a substantial amount of the water from the transition metal-exchanged ionomer without significantly affecting the physical or chemical properties of the ionomer. Preferably, the ionomer is dried under vacuum at room temperature for a time period sufficient to remove at least 50, preferably 80, more preferably 90, more preferably 95, and more preferably 99 percent of the water from the ionomer. When the ionomer has been exchanged with noble metal counterions, it is preferable to dry the ionomer in the absence of light to prevent significant photochemical reactions from occurring.

The dried ionomer can be formed into any of the conventional configurations which are operable for permselective applications. For example, the ionomer can be used in the form of flat sheets or self-supporting films, hollow fibers, and tubular structures. A sheet or film can be prepared via a variety of techniques which are disclosed in the Encyclopedia of Polymer Science and Technology, Vol. 6, page 764 (1967), including knife casting or dip casting an ionomer solution onto a glass surface or a supporting film, extrusion of an oriented layer or compression molding a layer of low orientation. Hollow fibers can be prepared by conventional hollow fiber spinning techniques. Tubular structures can be prepared by extrusion. Preferably, the ionomer is cast from solution to prepare an ultra-thin membrane in the form of a flat sheet or self-supporting film with a thickness as low as 0.3 um, preferably with a thickness between about 0.6 to about 40 um, and more preferably between about 0.6 to about 20 um.

The aliphatically unsaturated hydrocarbons which can be separated from a hydrocarbon mixture according to the method of this invention include paraffins and cycloparaffins, mono- and polyolefins having different complexing rates across the membrane, acetylenes, aromatics, and mixtures of these. The method of this invention is particularly useful for separating $C_{2-20}$ olefins, preferably $C_{2-8}$ olefins, e.g., ethylene, propylene, butenes, butadiene, isoprene, acetylene, and mixtures of these, from a mixture of paraffins containing at least one of these olefins.

The concentration of aliphatically unsaturated hydrocarbon in the mixture can vary over a wide range to achieve good separation, from as low as 2 weight percent to as high as 99 weight percent of the mixture.

For purposes of describing this invention, the first major surface of the membrane is a surface which contacts the feed stream, and the second major surface is the surface from which the desired permeate is discharged from the membrane.

The contacting of the hydrocarbon mixture with the ion exchange membrane of this invention and the subsequent withdrawal of the desired permeate therefrom can be carried out by using conventional membrane operating conditions, e.g., pervaporation or perstraction, at feed pressures up to 400 psig and at an operating pressure drop across the membrane desirably ranging from about 1 to about 3 atm. It is often advantageous to contact the second major surface of the membrane with a sweep gas or liquid to control membrane pressure drop and aid the removal of the desired permeate streams. The sweep gas or liquid selected should be inert with respect to the transition metal ions and should be easily removed from the permeate stream. Examples of suitable sweep gases include carbon dioxide, nitrogen, steam, methane and air. Examples of sweep liquids include paraffins having significantly different boiling points from that of the desired permeate.

In a preferred embodiment, membrane performance is enhanced by humidifying the hydrocarbon feed mixture or the sweep stream, if used. This can be accomplished by bubbling the feed mixture or sweep stream through water before it is contacted with the membrane.

The following examples are presented to illustrate the invention and are not intended to limit the scope of the claimed invention in any way. The term "propylene flux" is a measurement of the rate of propylene which permeates through the membrane. The "separation factor" is expressed as the ratio of propylene to propane in the permeate stream divided by the ratio of propylene to propane in the feed stream.

Example 1

Poly(phenylene oxide) is obtained from Poly-science, Inc., Warrington, Pennsylvania. The viscosity average molecular weight of this polymer is found to be 43,000 from its intrinsic viscosity in chloroform. Poly(phenylene oxide) (30.0 g) is dissolved in 600 ml of neutralized chloroform. Chlorsulfonic acid (9.8 g) is combined with 50 ml of chloroform. This is then added to the solvated polymer dropwise, with stirring, over a 20 minute period. The solution is stirred for a further 30 minutes. Two phases appear - a polymer phase and a chloroform phase. The polymer is separated from the chloroform, redissolved in methanol and recovered by evaporating off the methanol. The resulting sulfonated poly(phenylene oxide) (SPPO) is washed with distilled water then dried. Elemental analysis is used to confirm the degree of sulfonation as 2.5 meq/g. SPPO (30 g) is combined with 200 ml of deionized water and 12.8 g of silver nitrate and the mixture stirred at room temperature for 24 hours. The polymer is separated from the aqueous phase and washed with deionized water until the pH of the wash water exceeds 4.0. The resulting silver exchanged SPPO (Ag-SPPO) is dried under vacuum, at room temperature, in the dark for 3 days.

A 50 ml solution of 20% by weight Ag-SPPO in 3-butene-1-ol is prepared. This polymer solution is filtered once. A 10 mil casting knife is used to produce a thin film of Ag-SPPO which is placed in the dark at room temperature for 4 days to dry. The resulting film is 19 um thick [1]. This is placed in a laboratory diffusion cell. A 50/50 mixture of propane/ propylene is fed over one side of the film at 250 sccm and 30 psig. Before contacting the film, the feed stream is bubbled through distilled water. The other side of the film is swept with nitrogen at 250 sccm and 25 psig that also is been bubbled through distilled water. The film has the fo11owing separation characteristics:

[1] Thickness of film for each membrane prepared determined by scanning electron microscopy.

| Time After Start-up (Hours) | Propylene Flux (kg/m$^2$/d) | Separation Factor |
|---|---|---|
| 17 | 0.60 | 81 |
| 21 | 0.57 | 86 |
| 23 | 0.58 | 84 |
| 24 | 0.59 | 84 |

The results indicate that a self-supporting ultra-thin membrane prepared by the process of this invention exhibits an outstanding separation factor for the separation of propylene from a mixture of propane and propylene.

Comparative Example 1

A 50 ml solution of 20% by weight of the SPPO of Example 1 in dimethyl formamide is prepared. This polymer solution is filtered once. A 10 mil casting knife is used to produce a thin film of SPPO which is dried in an oven at 70°C for 16 hours. The dried SPPO film is 20 um thick. The SPPO film is then immersed in 2 M silver nitrate solution, filtered and washed with deionized water until the pH of the wash water exceeds 4. The resulting Ag-SPPO film is swept dry with nitrogen, at room temperature, in the dark for 3 days. The prepared film is placed in a laboratory diffusion cell and tested for its ability to separate propylene from a 50/50 mixture of propane/propylene under substantially identical conditions as the conditions described in Example 1. The film has the following separation characteristics:

| Time After Start-up (Hours) | Propylene Flux (kg/m$^2$/d) | Separation Factor |
|---|---|---|
| 2 | 11 | 1.3 |
| 3 | 12 | 1.2 |
| 4 | 14 | 1.1 |

The results indicate that a self-supporting ultra-thin membrane prepared by the prior art processes exhibit a very poor separation factor for the separation of propylene from a mixture of propane and propylene. In fact, this membrane is incapable of achieving any significant degree of separation at all.

Example 2

The polymer and casting solution is prepared substantially as in Example 1 with the following significant exception. The casting solution is poured on top of a pourous polyethylene ultrafiltration membrane sold commercially by Celanese Corp. as Celanaid 2500. The solution is allowed to wet the entire top surface of the Celgard membrane. The casting solution is then drained off by placing the Celgard membrane in a vertical position. After this polymer solution has dried, a thin film of Ag-SPPO is left covering the surface of the Celgard membrane. The film thickness is approximately 22 um. The membrane is tested under the conditions specified in Example 1. The film has the following separation characteristics:

| Time After Start-up (Hours) | Propylene Flux $(kg/m^2/d)$ | Separation Factor |
|---|---|---|
| 100 | 0.55 | 89 |
| 110 | 0.55 | 88 |
| 120 | 0.55 | 85 |

The results indicate that a significantly improved separation factor can be achieved by preparing a composite membrane using the process of this invention. The improved separation factor is achieved without a significant decrease in membrane flux.

**Claims**

1. A process for preparing a semipermeable ion exchange membrane comprising the steps of:

(a) contacting an ionomer with an aqueous solution of a transition metal salt under conditions sufficient to exchange a plurality of counterions associated with ionic groups of the ionomer for transition metal ions of the transition metal salt;

(b) drying the transition metal-exchanged ionomer thereby removing a substantial amount of water from the ionomer; and

(c) forming the dried ionomer into a configuration which is operable for permselective applications

2. The process of claim 1 wherein the ionomer is an aromatic polymer having ionic groups bound to a plurality of aromatic rings on the polymer.

3. The process of claim 2 wherein the aromatic polymer is functionalized with anionic groups capable of exchanging cations.

4. The process of claim 2 or claim 3 wherein the ionically bound groups are sulfonate, carboxylate, or phosphonate groups.

5. The process of any of claims 1 to 4 wherein the ionomer is derived from poly(phenylene oxide), polystyrene, polyacrylic acid, or a perfluorinated polymer.

6. The process of any of claims 1 to 5 wherein the ionomer has an ion exchange capacity of between about 2.0 and 3.5 meq/g.

7. The process of any of claims 1 to 6 wherein the transition metal ions are derived from a noble metal.

8. The process of claim 7 wherein the noble metal is silver.

9. The use of a semipermeable ion exchange membrane prepared by the process of any of claims 1 to 8 in permselective applications.

10. A method of separating an unsaturated and aliphatically unsaturated hydrocarbon from a hydrocarbon mixture comprising the steps of:

(a) contacting a first major surface of the semi-permeable ion exchange membrane prepared by the process of any of claims 1 to 9 with the hydrocarbon mixture; and

(b) withdrawing from a second major surface of the membrane a permeate with an increased concentration of the aliphatically unsaturated hydrocarbon relative to the concentration of the aliphatically unsaturated hydrocarbon in the hydrocarbon mixture.

11. The method of claim 10 wherein the aliphatically unsaturated hydrocarbon is a $C_{2-20}$ olefin or a mixture of $C_{2-20}$ olefins.

12. The method of claim 11 wherein the aliphatically unsaturated hydrocarbon is ethylene, propylene, a butene, butadiene, isoprene, acetylene, or a mixture of two or more of these.

13. The method of any of claims 10 to 12 wherein the second major surface is contacted with a sweep case or liquid thereby aiding the removal of the desired permeate.

14. The method of claim 13 wherein the hydrocarbon mixture or the sweep gas or liquid is humidified by bubbling the hydrocarbon feed mixture or sweep gas or liquid through water before it is contacted with the membrane.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | US-A-4 789 386  (W.L. VAUGHN) <br> * Especially column 4, line 56 - column 6, line 33 * <br> --- | | B 01 D  69/14 <br> B 01 D  71/82 <br> C 07 C   7/144 |
| A | GB-A-2 169 300  (MONTSANTO CO.) <br> * Especially page 4, lines 1-4 * <br> --- | | |
| A | US-A-4 741 744  (M.L. WU) <br> * Especially column 5, lines 49-60 * <br> --- | | |
| A | GB-A-1 434 639  (MONTSANTO CO.) <br> * Especially page 6, lines 76-88 * <br> --- | | |
| A | US-A-3 773 844  (E. PERRY) <br> --- | | |
| P,A | INDUSTRIAL AND ENGINEERING CHEMISTRY RESEARCH, vol. 28, no. 7, July 1989, pages 1020-1024; C.A. KOVAL: "Styrene/ethylbenzene separation using facilitated transport through perfluorosulfonate ionomer membranes" <br> ----- | | |

TECHNICAL FIELDS
SEARCHED (Int. Cl.5)

B 01 D  69/00
B 01 D  71/00
C 07 C   7/00

The present search report has been drawn up for all claims

| Place of search | Date of completion of the search | Examiner |
|---|---|---|
| THE HAGUE | 28-09-1990 | DEVISME F.R. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P0401)